# EUROPEAN PATENT SPECIFICATION

(11) **EP 2 371 411 B1**
(45) Date of publication and mention of the grant of the patent: **31.10.2012**
(21) Application number: 10158201.3
(22) Date of filing: 29.03.2010
(51) Int. Cl.: A61M 16/00

(54) **Visual status indicator for patient circuit**
Visuelle Statusanzeige für eine Patientenschaltung
Indicateur d'état visuel pour tubulure patient

(43) Date of publication of application: 05.10.2011
(73) Proprietor: Dräger Medical GmbH, 23558 Lübeck (DE)
(72) Inventor: Van Dieten, Jasper, 5651, EA Eindhoven (NL); Blonet, Jasper, 4105, SB Culemborg (NL)
(74) Representative: UEXKÜLL & STOLBERG

(56) References cited:
- US-A1- 2003 234 015
- US-A1- 2005 011 282
- US-A1- 2006 107 745

## Description

### FIELD OF THE INVENTION

The invention relates to a visual status indicator for providing a status indication of a fluid transported through a patient circuit between a medical device and a patient. The invention further relates to a respirator hose comprising the visual status indicator, and to a medical device for supplying a status signal to the visual status indicator.

### BACKGROUND OF THE INVENTION

Mechanical ventilators are used in modern medicine to mechanically assist or replace spontaneous breathing of a patient. For that purpose, the mechanical ventilator may be connected to the patient by means of a patient circuit and supply or retrieve a fluid from the patient through the patient circuit. The patient circuit typically comprises a conduit for transporting the fluid, and is connected to the patient through e.g. the mouth or the nose.

Mechanical ventilators normally supply or retrieve a gas, e.g. inspiratory and expiratory air. Additionally, medical devices exist that, instead of a gas, supply or retrieve a liquid. Examples of such devices are extracorporeal blood pumps, liquid ventilators and dialysis machines.

It is desirable to provide a medical caretaker of the patient or a medical operator of the mechanical ventilator (henceforth commonly referred to as caretaker) with information about the status of relevant parameters of the fluid being supplied to or retrieved from the patient through the patient circuit.

A known method for providing the caretaker with said status information is to display the information on a display that is part of a ventilator system. More specifically, US 6,675,801 discloses a graphic user interface for the display of patient data and ventilator status. The user interface includes a graphic representation of a breath cycle that displays the breath cycle currently being ventilated, and is connected to a breath delivery part of the ventilator system.

US 2005/0011282 A1 discloses a visual status indicator for providing a status indication of a fluid transported through a patient circuit between a medical device and a patient. The visual status indicator comprises a fixing means for being fixed to the patient circuit and a light source which is configured to provide a light signal in dependence on a measured status of the fluid transported through the patient circuit. Further, the patient circuit comprises a respirator hose. The visual status indicator is provided with a housing having an inlet and an outlet so as to allow fluid to flow from the inlet to the outlet through the status indicator housing. Thus, the visual status indicator forms an integral part of the patient circuit.

US 2006/0107745 A1 describes a visual status indicator for providing a status indication of a fluid transported through a patient circuit and having an fixing means for fixing to the patients circuit and a plurality of light sources for providing a light signal in dependence on a measured status of a fluid transported through the patient circuit. The visual status indicator comprises a housing which is on one end connected to the patient circuit by the fixing means.

### SUMMARY OF THE INVENTION

A problem of the above graphic user interface is that the caretaker of the patient has to specifically look at the ventilator system to obtain status information concerning the fluid transported through the patient circuit. However, the caretaker is naturally inclined to directly look at the patient during observation, and in particular while actively treating the patient. Despite this, the caretaker thus as to additionally look at the ventilator system to obtain status information that cannot be obtained by merely looking at the patient.

Consequently, it is inconvenient for the caretaker to obtain status information concerning the fluid transported through the patient circuit, since he has to specifically pay attention to the ventilator system. Additionally, having to obtain status information from the ventilator system can pose a risk to the patient, since the caretaker may be so focused on the patient that he neglects the ventilator system and thus fails to obtain status information that may be relevant in the treatment of the patient. Furthermore, the caretaker may not be able to see the ventilator system or its user interface due to its placement.

It would be advantageous to have an improved device for providing status information to a patient's caretaker. To better address this concern, the invention provides a visual status indicator for providing a status indication of a fluid transported through a patient circuit between a medical device and a patient, the visual status indicator comprising an affixing means for affixing the visual status indicator along a portion of the patient circuit, and a light source for providing at said portion a light signal in dependence on a measured status of the fluid transported through the patient circuit, the patient circuit comprising a respirator hose. The affixing means comprises a sleeve for fitting at least partially around said respirator hose.

The visual status indicator provides along the patient circuit a visual status indication of the fluid transported through said circuit. The patient circuit is inherently close to the patient, since it supplies fluids to or retrieves fluids from the patient. In fact, a portion of the patient circuit, such as a breathing mask, has direct contact with the patient. Hence, by affixing the visual status indicator along a portion of the patient circuit, the visual status indication may be provided nearby the patient.

A visual status indicator nearby the patient allows the caretaker of the patient to see the visual status indicator when observing the patient. Consequently, it is convenient for the caretaker to obtain the status information since he can obtain said information while naturally paying attention to the patient. Furthermore, the risk for the patient may be lowered, since even if the caretaker is focused on the patient instead of the medical device, he can still obtain relevant status information as the visual status indicator is located nearby the patient.

In an embodiment, the light source is arranged for varying a visual characteristic of the light signal in dependence on the measured status of the fluid, the visual characteristic comprising at least one of: color, intensity and position of the light signal.

The color, intensity and position of a light signal are clearly noticeable to an observer or the caretaker. Hence, by varying specifically those characteristics of the light signal, it is ensured that the caretaker will clearly notice the light signal. Furthermore, the risk that the caretaker fails to notice the visual status indicator may be reduced.

In an embodiment, the measured status of the fluid comprises at least one of: flow rate, flow direction, change in flow rate, change in flow direction, pressure, fluid composition and fluid concentration.

By providing the status of these parameters, the visual signal indicator is able to provide relevant status information to the caretaker.

In an embodiment, the visual status indicator comprises a status input for receiving from the medical device a status signal representative of the measured status of the fluid, and the light source is arranged for providing the light signal in dependence on the status signal.

The status input enables the visual status indicator to obtain a signal representative of the measured status from the medical device. It is therefore not necessary for the visual status indicator to comprise an integrated measurement means, as the measured status may be obtained from a status output of the medical device. Hence, the visual status indicator may be reduced in cost and complexity. Furthermore, the medical device may already provide status information on, for example, a display. The same information may now be additionally shown along a portion of the patient circuit.

In an embodiment, the status input of the visual status indicator is arranged for forming, together with a fluid connector of the patient circuit, a connector that allows the visual status indicator and the patient circuit to be simultaneously connected to the medical device.

In a related embodiment, a medical device for transporting a fluid through a patient circuit comprises a device connector that allows the visual status indicator and patient circuit to be simultaneously connected to the medical device, the device connector comprising a fluid connector for providing the fluid to the patient circuit and a status output for providing to the visual status indicator a status signal representative of a measured status of the fluid.

The patient circuit is connected to the medical device through a fluid connection when transporting fluid between the medical device and the patient. For this purpose, the patient circuit may comprise a fluid connector. By having the status input of the visual status indicator and the fluid connector form a single connector, the visual status indicator can be connected to the medical device together with establishing the fluid connection.

For this purpose, the medical device may comprise a device connector that comprises a status output and a fluid connector. The status output can thus be connected to the status input of the visual status indicator when connecting the fluid connector of the patient circuit to the fluid connector of the medical device. Hence, it is not required to additionally connect the visual status indicator to the medical device. The visual status indicator may therefore be easier to handle for caretakers.

In an embodiment, the status signal comprises an electrical signal, and the light source is arranged for generating the light signal using the electrical signal.

An electrical signal can be used for efficiently transmitting information, since electrical signals provide high bandwidths and allow robust transmission over large distances. The electrical signal thus enables the medical device to provide the information for varying the visual characteristics of the light signal in an efficient manner.

In an embodiment, the status signal comprises a light signal, and the light source is arranged for emitting the light signal.

This way, the visual status indicator only needs to emit the light signal supplied from an external source, e.g. a light source integrated in the medical device. Consequently, the visual status indicator may be free of electrical signals and parts, and thus be reduced in cost and complexity.

In an embodiment, the status input comprises a first portion of a light conductor, the light source comprising a second portion of the light conductor, the light signal being transported from the first portion to the second portion of the light conductor and the light signal being emitted out of the second portion.

A light conductor may transport light from one portion, i.e. an entry point, of the light conductor to another portion, i.e. an exit point. The light conductor can thus be used in the visual status indicator to transport the light signal from the status input to the light source. Additionally, the respective portions of the light conductor may be directly used as status input and light source of the visual status indicator. Consequently, the visual status indicator may be lower in cost and complexity as fewer components are needed.

In an embodiment, the measured status is associated with flow rate or flow direction of the fluid, and the light source is arranged for varying, in dependence on the flow rate or the flow direction, a position of the light signal to create a moving visual status indication of the flow rate or the flow direction.

The flow of a fluid relates to the movement, i.e. the change in position, of the fluid. The status of the fluid's movement can be intuitively visualized by moving the light signal. The light signal can be moved by actually changing the position of the light source, i.e. by moving the light source, or by sequentially providing a light signal at different positions to create an appearance of a moving light source. To that end, the visual status indicator may comprise multiple light sources.

The direction of the light signal's movement may reflect the flow direction, and the speed of the light signal's movement may be proportional to the flow rate. Furthermore, the light signal's movement may be repeated once it cannot be continued, e.g. once it reaches an end of the visual status indicator. The caretaker thus may be able to obtain said status information in an intuitive manner that requires little interpretation effort.

In an embodiment, the measured status is associated with composition, concentration or pressure of the fluid, and the light source is arranged for varying, in dependence on the measured status, a color or an intensity of the light signal.

Next to the flow rate and flow direction, the composition, concentration and pressure are important characteristics of the fluid. Of these characteristics, a certain range of values may be of particular interest. This range can be intuitively visualized by varying the color of the light signal within a range of colors, or the intensity of the light signal within a range of intensities. A combination of both may be equally possible.

In an embodiment, the affixing means is arranged for affixing the visual status indicator at least along a portion of the patient circuit that is nearer to the patient than to the medical device.

By allowing the visual status indicator to be affixed, or by affixing it, along a portion of the patient circuit that is nearer to the patient than to the medical device, the visibility of the visual status indicator, and thus the light signal, is improved, as the caretaker is naturally inclined to look at the patient rather than the medical device.

According to the invention, the portion of the patient circuit comprises a respirator hose and the affixing means comprises a sleeve for fitting at least partially around the respirator hose.

A patient circuit typically comprises a respirator hose for transporting the fluid. The visual status indicator may be easily affixed to the patient circuit by using a sleeve to fit around the respirator hose. If the sleeve is fitted around the respirator hose, the visual status indicator and the respirator hose effectively form a single unit that may be easy to handle. Furthermore, the sleeve may be easily detached from the respirator hose, e.g. to allow replacement or cleaning of the respirator hose.

In an embodiment, a respirator hose for transporting a fluid between a medical device and a patient comprises the visual status indicator.

A patient circuit may comprise a respirator hose for transporting fluid. A respirator hose that comprises the visual status indicator can provide a visual status indication on its own, i.e. without requiring a separate visual status indicator. The respirator hose may also be easier to handle by caretakers than a separate respirator hose and a separate visual status indicator, as e.g. the step of connecting the visual status indicator to the respirator hose may be omitted.

### BRIEF DESCRIPTION OF THE DRAWINGS

These and other aspects of the invention are apparent from and will be elucidated with reference to the embodiments described hereinafter. In the drawings,
- Fig. 1: shows a visual status indicator affixed along a portion of a patient circuit;
- Fig. 2: shows a visual status indicator comprising a light conductor;
- Fig. 3: shows a visual status indicator comprising clamps as affixing means;
- Fig. 4: shows a visual status indicator comprising a sleeve as affixing means;
- Fig. 5A, 5B and 5C: show consecutive movement phases of a moving visual status indication of flow direction;
- Fig. 6: shows a visual status indicator that is an integral part of a patient circuit.

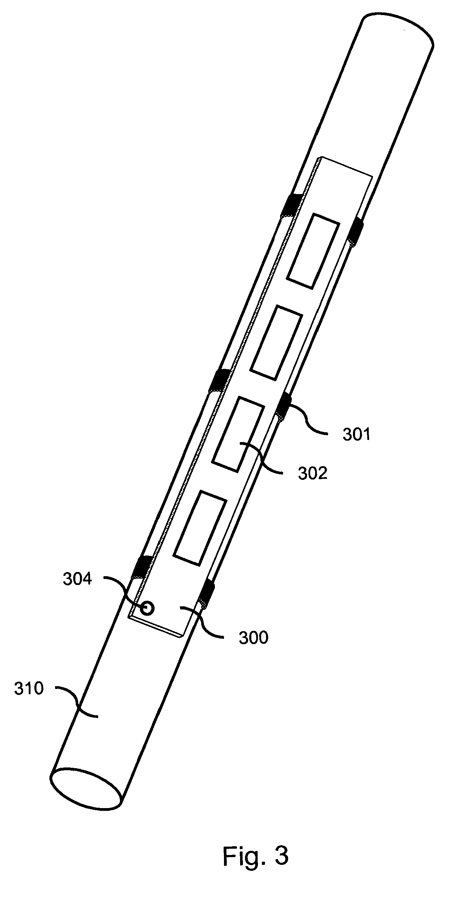

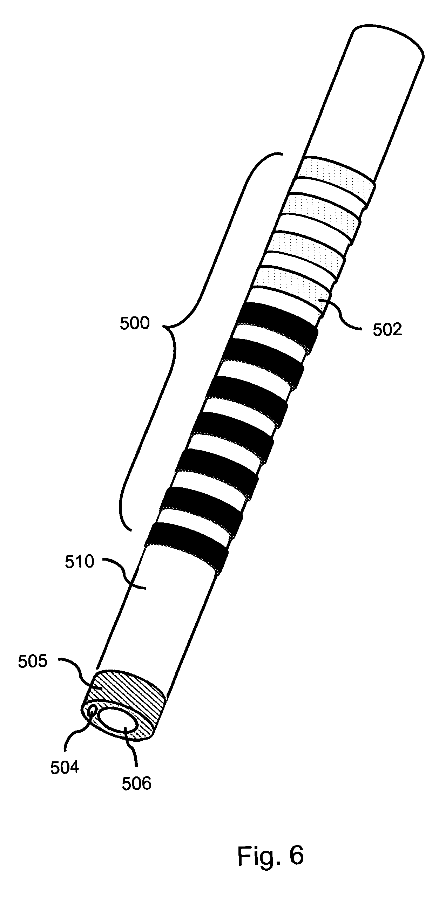

The embodiments of figs. 3 and 6 are given as examples and do not form part of the claimed matter.

In the drawings, similar items are denoted by the same reference numerals.

### DETAILED DESCRIPTION OF EMBODIMENTS

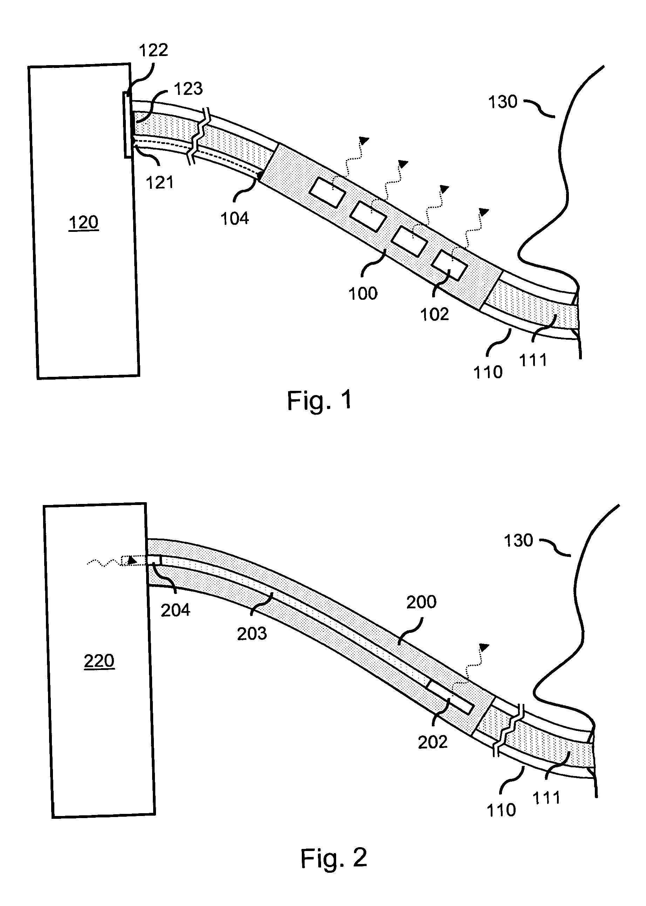

Fig. 1 shows a visual status indicator 100 that is affixed along a portion of a patient circuit 110, with the patient circuit being shown in the form of its cross-section. The patient circuit 110 connects a medical device 120 to a patient 130, and is arranged for transporting a fluid 111 between the medical device 120 and the patient 130. Typically, the patient circuit 110 comprises a conduit for transporting the fluid 111. For example, the patient circuit 110 may comprise a hose or a coaxial hose. The patient circuit 110 may also solely consist of a hose. Furthermore, the patient circuit 110 may comprise an endotracheal tube or breathing mask for connecting to the patient 130.

The medical device 120 may be a respiratory device or mechanical ventilator. In this case, the fluid 111 transported by the patient circuit 110 is usually a breathing gas such as air or oxygen. Although not shown in Fig. 1, the medical device 120 may also be an extracorporeal blood pump, dialysis machine or similar device. In this case, the transported fluid 111 is a liquid such as blood, and the patient circuit typically connects to the patient through an intravenous cannula or a similar tube.

The portion of the patient circuit 110 to which the visual status indicator 100 is affixed may be any portion of the patient circuit. It is generally desirable, however, that the caretaker is able to see the visual status indicator 100 when looking at the patient 130. The chance of the caretaker seeing the visual status indicator 100 is increased when the visual status indicator 100 is affixed along at least a portion of the patient circuit 110 that is nearby the patient. For that purpose, the visual status indicator 100 may be affixed in its entirety along a portion of the patient circuit 110 nearby the patient 130, or may be affixed such that at least a portion of the visual status indicator 100 is located nearby the patient 130.

The visual status indicator 100 comprises a light source 102 for providing a light signal in dependence on a measured status of the fluid 111. The measured status may comprise any status that is relevant for the caretaker of the patient. For example, if a patient is mechanically ventilated using air, the air pressure and the O₂ and CO₂ concentrations of the air may be of particular relevance to the caretaker. Generally, relevant statuses for the caretaker may include the flow rate, flow direction, change in flow rate, change in flow direction, pressure, fluid composition and fluid concentration.

The light source 102 of the visual status indicator 100 may be any type of light source that is suitable for usage in the visual status indicator 100. For example, the light source 102 may be a Light-Emitting Diode (LED). LED's have the advantage that they are small, robust, energy efficient and operate at low voltages. The latter aspect may be especially relevant in medical devices, where a light source with a high operating voltage may increase the risk of electrical shock to the patient in case of a malfunction. The light source 102 may further be capable of producing multiple colors as well as only a single color.

The light signal generated by the light source 102 has a visual characteristic that is largely determined by the light source 102. As such, the visual characteristic may be varied by the light source 102 in dependence on the measured status of the fluid 111. Most light sources are capable of varying at least the intensity of the light signal. As such, it is possible to map a range of intensities of the light signal to a range of the measured status. For example, a low O₂ level may result in a high intensity of the light signal, whereas an optimal O₂ level may result in a low intensity of the light signal. The intensity may also be used in other ways to indicate the measured status. For example, blinking or any other means of time-domain modulation of the intensity of the light signal may be used.

The visual characteristic being varied may also be the color of the light signal.
For example, the color of the light signal may varied between green, orange and red to indicate an O₂ level ranging from optimal to dangerously low. The color of the light signal can also be combined with the intensity to more clearly indicate the measured status. For example, a too high CO₂ level may be indicated by a high-intensity blinking red light signal, whereas an optimal CO₂ level may be indicated by a low-intensity constant green light signal.

The visual characteristic being varied may also be the position of the light signal. For that purpose, the light source 102 may be arranged to physically change its position. Alternatively, multiple light sources may be used that sequentially provide the light signal at varying positions. For example, the visual status indicator 100 may comprise four light sources, and the light signal may be provided by the light source nearest to the patient to signal an optimal O₂ level. Similarly, the light source nearest to the medical device may signal a dangerous O₂ level. The light signal may also comprise a display, and the position of the light signal may be varied by suitably addressing and driving the display.

The measured status may be received from an external source such as the medical device 120 by means of a status signal representative of the measured status of the fluid 111. For that purpose, the visual status indicator 100 may comprise a status input 104 for receiving the status signal, and the medical device 120 may comprise a status output 121 for providing the status signal. In case the status signal is an electrical signal, the status input 104 may be connected to the status output 121 by means of an electrical connector such as a conductive wire.

The electrical connector may be entirely separated from the patient circuit 110, which may allow easy removal of the electrical connector in case the visual status indicator 100 is removed from the patient circuit 110. The electrical connector may also be affixed on or within the patient circuit 110. This may increase the ease of handling of the patient circuit 110 for the caretaker. The status output 121 of the medical device 120 may also be located nearby the fluid connection between the medical device 120 and the patient circuit 110, such that, when attaching the patient circuit 110 to the medical device 120, the electrical connection is established simultaneously with the fluid connection. For that purpose, the fluid connection and the electrical connection may also be established by a single connector connecting the patient circuit 110 with the medical device 120. In this case, the medical device may comprise a device connector 122 for connecting to the patient circuit's connector, with the device connector comprising the status output 121 and a fluid connector 123.

Fig. 2 also shows a visual status indicator 200 that is affixed along a portion of a patient circuit 110, with the patient circuit being shown in the form of its cross-section. However, in contrast to the visual status indicator 100 shown in Fig. 1, the visual status indicator 200 now comprises as light source 202 a portion of a light conductor 203. The light conductor 203 is arranged for receiving from the medical device 220 a light signal via a first portion 204 of the light conductor 203, conducting the light signal from the first portion 204 to the second portion 202, and emitting the light signal from the second portion 202. The status signal is thus a light signal generated by the medical device 220.

The light conductor 203 may comprise an optical fiber. Alternatively, the light conductor 203 may also comprise a light conductive flexible panel comprising a reflective plane and a light-emitting plane above the reflective plane. In such a light conductive flexible panel, the light enters the light conductor in between both planes, and is emitted out of the light-emitting plane across its entire surface. As such, the light conductor may act as a light source 102 with its entire surface, allowing for the light signal to be provided along a large portion of the patient circuit 110. Furthermore, the light conductor may be directly affixed to the patient circuit 210 by e.g. gluing the light conductor to the patient circuit's surface.

Fig. 3 shows a visual status indicator 300 that is affixed along a portion of the patient circuit 310 by means of an affixing means 301. Here, only a part of the patient circuit is shown, as the patient circuit typically runs further in both directions. The affixing means 301 is shown to consist of three clamps that affix the visual status indicator 300 to the patient circuit. The visual status indicator 300 may be permanently affixed to the patient circuit 310. This may be the case if the visual status indicator 300 is clamped to the patient circuit 310 during manufacturing of said circuit using clamps that cannot be readily de-clamped.

However, the clamps 301 may also allow the visual status indicator 300 to be freely attached and detached to and from the patient circuit 310. This may allow a health care facility to use a standard patient circuit, and provide it with the ability of providing a visual status indication by attaching the visual status indicator 300. Similarly, the clamps 301 may allow the visual status indicator 300 to be removed during cleaning of the patient circuit 310 to prevent damage to the visual status indicator 300. Also, the patient circuit 310 may be a disposable patient circuit. In this case, the visual status indicator 300 can be re-used by detaching it from a used patient circuit and attaching it to a new patient circuit.

In the example of Fig. 3, the visual status indicator 300 comprises four individual light sources 302 that are arranged for varying, in dependence on composition, concentration or pressure of the fluid, a color or an intensity of the light signal. The fluid's composition, concentration or pressure may be received as a status signal from the medical device through the status input 304. The status signal is well suited to be visualized by color or intensity. For example, the O₂ and CO₂ concentrations as well as the air pressure may each be visualized by a separate light source by means of varying the color of the light signal between red and green. The fourth light source may then be used to indicate a possible malfunctioning of the visual status indicator 300 by means of a blinking red light signal.

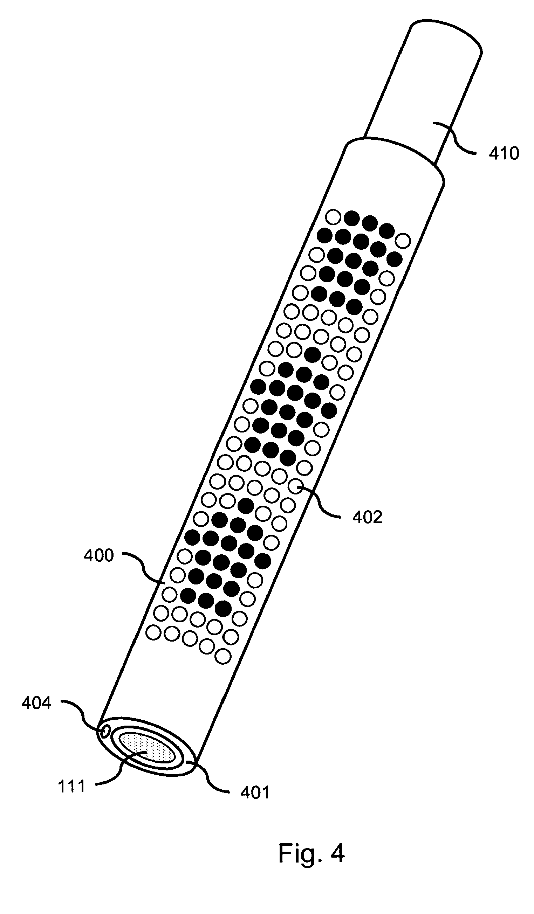

Fig. 4 shows a visual status indicator 400 comprising a sleeve 401 as affixing means, and a patient circuit comprising a respirator hose 410 for transporting the fluid 111. The sleeve 401 is arranged for fitting at least partially around the respirator hose 410 so as to hold the visual status indicator 400 in place. Similarly to the clamps shown in Fig. 3, the sleeve may be permanently affixed to the respirator hose 410, or may be freely attachable to and detachable from the respirator hose 410. In case the sleeve 401 is permanently affixed to the respirator hose 410, the visual status indicator 400 may form an integral part of the respirator hose 410. The patient circuit may thus be directly manufactured with the ability to provide a visual status indication.

In the example of Fig. 4, the sleeve 401 is shown to extend entirely towards one end of the respirator hose 410. Although not shown in Fig. 4, this end of the respirator hose 410 can connect to the medical device, whereas the opposite end further extends towards the patient. For the purpose of connecting the visual status indicator 400 to the medical device, the sleeve 401 comprises a status input 404. The status input 404 is located nearby the respirator hose 410, such that when connecting the respirator hose 410 to the medical device, the status input 404 simultaneously establishes a connection with a corresponding status output of the medical device. For that purpose, the status input 404 may be a socket, and the status output of the medical device may be the corresponding plug.

The visual status indicator 400 further comprises a 2-dimensional array of individual light sources 402. This allows the visual status indicator 400 to represent characters, symbols and images by means of suitable addressing each light source 402. Fig. 4 shows an example where the array of light sources 402 are used to signal the flow direction of the fluid 111 with arrows pointing in said direction. For reason of clarity, in this and subsequent drawings a black light source indicates the light source generating a light signal, whereas a white light source indicates the light source not generating a light signal.

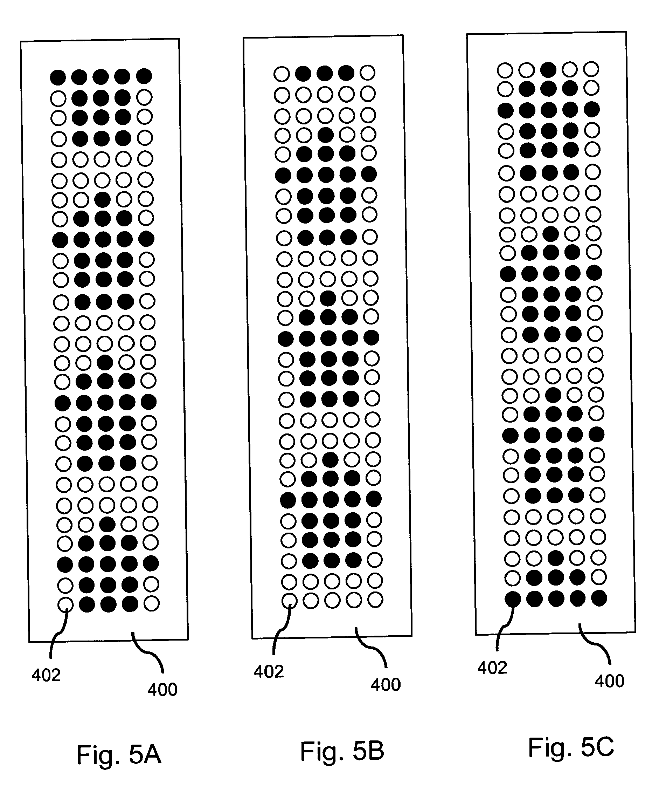

Fig. 5A, Fig. 5B and Fig. 5C show the 2-dimensional array of light sources 402 generating a moving visual status indication. Fig. 5A shows the 2-dimension array of light sources producing a pattern depicting multiple arrows. Fig. 5B shows the 2-dimensional array of light sources at a later instance in time, in which the pattern has moved in the direction of the arrows. Finally, Fig. 5C shows the 2-dimensional array of light sources at an again later instance in time, in which the pattern has moved even further in the direction of the arrows. The visual status indicator 400 can thus be used to generate a pattern moving in the flow direction of the fluid, thereby creating an intuitive indication of said flow direction.

The moving visual status indication may also be used to indicate the flow rate of the fluid. For example, a slowly moving pattern may indicate a low flow rate, whereas a fast moving pattern may indicate a high flow rate. The movement of the pattern may also be combined with a varying color of the pattern to provide an even clearer visual status indication. For example, if the flow rate is dangerously low, the pattern may be shown in red, whereas in case of an optimum flow rate, the pattern may be shown in green. Various other patterns are equally possible. For example, instead of arrows, the visual status indicator 400 may be used to display moving or non-moving text or symbols.

Fig. 6 shows a visual status indicator 500 that is an integral part of a patient circuit. In this example, the patient circuit comprises a respirator hose 510, and the visual status indicator 500 consists of a series of ring-shaped light sources 502. The visual status indicator 500 can form an integral part of the respirator hose 510 in a variety of ways. For example, the light sources 502 may be bonded to the surface of the respirator hose 510. The light sources 502 may be also integrated inside the respirator hose's outer wall. Furthermore, any electronic circuits and wiring required to power the light sources 502 may be integrated within the respirator hose 510 as well.

The patient circuit further comprises a connector 505 that can be used to establish a connection with the medical device. The connector 505 comprises an electrical connector 504 and a fluid connector 506 for receiving from the medical device an electrical signal and the fluid, respectively. The electrical signal may provide the measured status as well as electrical energy to the visual status indicator 500. Although not shown in Fig. 6, the medical device may comprise a device connector to receive the patient circuit's connector. Consequently, when connecting the patient circuit to the medical device, the connector 505 establishes both a fluid connection as well as a connection for receiving the measured status.

The light sources 502 may be used to provide a moving status indication that is similar to that provided by the visual status indicator 400 shown in Fig. 4. However, instead of showing arrows, the flow direction may be indicated by one or multiple light signals moving along said flow direction. The series of light sources 502 may also be used to display non-moving patterns. For example, the O₂ concentration may be indicated by providing a number of light signals along the patient circuit that is proportional to the O₂ concentration. In this example, an optimal O₂ concentration may be indicated by having all light sources 502 emit a light signal, whereas a O₂ concentration that is half the optimal value may be indicated by having only half the light sources 502 emit a light signal.

Although not shown in Fig. 6, the respirator hose 510 may further comprise a measurement device for measuring a status of the fluid transported within the respirator hose 510. The measurement device may, for example, be a pressure sensor or O₂ concentration sensor. It may also be able to simultaneously measure multiple statuses of the fluid. This allows the respirator hose 510 to directly provide the measured status to the visual status indicator 500. Hence, the medical device may not need to provide the measured status.

It should be noted that the corresponding technical features of the visual status indicators described in the above embodiments can be freely interchanged. For example, the 2-dimensional array of light sources 402 shown in Fig. 4 may be used in the visual status indicator 300 shown in Fig. 3 instead of the four individual light sources 302. As such, a visual status indicator is obtained that comprises clamps 301 as affixing means as well as a 2-dimensional array of light sources 402. Furthermore, the shape and exact placement of the light sources in the drawings is merely illustrative, as light sources exist in a wide range of shapes and typically allow a wide range of placements.

It should be further noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. Visual status indicator (100, 200, 300, 400, 500) for providing a status indication of a fluid (111) transported through a patient circuit (110, 310, 410, 510) between a medical device (120, 220) and a patient (130), the visual status indicator comprising:
- an affixing means (301, 401) for affixing the visual status indicator along a portion of the patient circuit, and
- a light source (102, 202, 302, 402, 502) for providing at said portion a light signal in dependence on a measured status of the fluid transported through the patient circuit,
wherein a portion of the patient circuit comprises a respirator hose (410),
**characterized in that**
the affixing means comprises a sleeve (401) for fitting at least partially around the respirator hose.

2. Visual status indicator (100, 200, 300, 400, 500) according to claim 1, wherein the light source (102, 202, 302, 402, 502) is arranged for varying a visual characteristic of the light signal in dependence on the measured status of the fluid (111), the visual characteristic comprising at least one of: color, intensity and position of the light signal.

3. Visual status indicator (100, 200, 300, 400, 500) according to claim 1 or 2, wherein the measured status of the fluid (111) comprises at least one of: flow rate, flow direction, change in flow rate, change in flow direction, pressure, fluid composition and fluid concentration.

4. Visual status indicator (100, 200, 300, 400, 500) according to any one of the above claims, the visual status indicator comprising a status input (104, 204, 304, 404, 504) for receiving from the medical device (120, 220) a status signal representative of the measured status of the fluid (111), and the light source (102, 202, 302, 402, 502) being arranged for providing the light signal in dependence on the status signal.

5. Visual status indicator (100, 200, 400, 500) according to claim 4, wherein the status input (104, 204, 404, 504) of the visual status indicator is arranged for forming, together with a fluid connector (506) of the patient circuit, a connector (505) that allows the visual status indicator and the patient circuit to be simultaneously connected to the medical device.

6. Visual status indicator (100, 300, 400, 500) according to claim 4 or 5, wherein the status signal comprises an electrical signal, and the light source (102, 302, 402, 502) is arranged for generating the light signal using the electrical signal.

7. Visual status indicator (200) according to claim 4 or 5, wherein the status signal comprises a light signal, and the light source (202) is arranged for emitting the light signal.

8. Visual status indicator (200) according claim 7, wherein the status input comprises a first portion (204) of a light conductor (203), the light source comprising a second portion (202) of the light conductor, the light signal being transported from the first portion to the second portion of the light conductor and the light signal being emitted out of the second portion.

9. Visual status indicator (400, 500) according to any one of the above claims, wherein the measured status is associated with flow rate or flow direction of the fluid (111), and the light source (402, 502) is arranged for varying, in dependence on the flow rate or the flow direction, a position of the light signal to create a moving visual status indication of the flow rate or the flow direction.

10. Visual status indicator (100, 200, 300) according to any one of claims 1 to 8, wherein the measured status is associated with composition, concentration or pressure of the fluid (111), and the light source (102, 202, 302) is arranged for varying, in dependence on the measured status, a color or an intensity of the light signal.

11. Visual status indicator (300, 400) according to any one of the above claims, wherein the affixing means (301, 401) is arranged for affixing the visual status indicator (300, 400) at least along a portion of the patient circuit (310, 410) that is nearer to the patient (130) than to the medical device (120).

12. Respirator hose (410, 510) for transporting a fluid (111) between a medical device (120) and a patient (130), the respirator hose comprising the visual status indicator of any one of the above claims.

13. Respirator hose (410, 510) according to claim 12, wherein the visual status indicator (400, 500) is an integral part of the respirator hose.

14. Medical device (120) for transporting a fluid (111) through a patient circuit (110), the medical device comprising a device connector (122) that allows the patient circuit and the visual status indicator of claim 5 to be simultaneously connected to the medical device, and the device connector comprising a fluid connector (123) for providing the fluid to the patient circuit and a status output (121) for providing to the visual status indicator (100) a status signal representative of a measured status of the fluid (111).

## Patentansprüche

1. Visueller Statusanzeiger (100, 200, 300, 400, 500) zum Liefern einer Statusanzeige eines Fluids (111), das durch eine Patientenleitung (110, 210, 310, 410, 510) zwischen einem medizinischen Gerät (120, 220) und einem Patienten (130) fließt, wobei der visuelle Statusanzeiger aufweist:
Befestigungsmittel (301, 401) zum Befestigen des visuellen Statusanzeigers entlang eines Bereichs der Patientenleitung und
eine Lichtquelle (102, 202, 302, 402, 502), um in dem Bereich ein Lichtsignal in Abhängigkeit von einem gemessenen Status des durch die Patientenleitung transportierten Fluids bereitzustellen,
wobei ein Bereich der Patientenleitung einen Beatmungsschlauch (410) aufweist,
**dadurch gekennzeichnet, dass**
die Befestigungsmittel eine Manschette (401) zum Anliegen wenigstens teilweise um den Beatmungsschlauch herum aufweisen.

2. Visueller Statusanzeiger (100, 200, 300, 400, 500) nach Anspruch 1, wobei die Lichtquelle (102, 202, 302, 402, 502) dazu eingerichtet ist, eine visuelle Eigenschaft des Lichtsignals in Abhängigkeit von dem gemessenen Status des Fluids (111) zu variieren, wobei die visuelle Eigenschaft wenigstens eines aufweist von: Farbe, Intensität und Position des Lichtsignals.

3. Visueller Statusanzeiger (100, 200, 300, 400, 500) nach Anspruch 1 oder 2, wobei der gemessene Status des Fluids (111) wenigstens eines aufweist von: Durchflussvolumenstrom, Volumenstromrichtung, Veränderung im Durchflussvolumenstrom, Veränderung in der Volumenstromrichtung, Druck, Fluidzusammensetzung und Fluidkonzentration.

4. Visueller Statusanzeiger (100, 200, 300, 400, 500) nach einem der vorhergehenden Ansprüche, wobei der visuelle Statusanzeiger einen Statuseingang (104, 204, 304, 404, 504) aufweist, um von dem medizinischen Gerät (120, 220) ein Statussignal zu empfangen, das repräsentativ für den gemessenen Status des Fluids (111) ist, und wobei die Lichtquelle (102, 202, 302, 402, 502) dazu eingerichtet ist, ein Lichtsignal in Abhängigkeit von dem Statussignal bereitzustellen.

5. Visueller Statusanzeiger (100, 200, 300, 400, 500) nach Anspruch 4, wobei der Statuseingang (104, 204, 404, 504) des visuellen Statusanzeigers dazu ausgestaltet ist, um, zusammen mit einem Fluidverbinder (506) der Patientenleitung, einen Verbinder (505) zu bilden, der es erlaubt, dass der visuelle Statusanzeiger und die Patientenleitung gleichzeitig mit dem medizinischen Gerät verbunden sind.

6. Visueller Statusanzeiger (100, 300, 400, 500) nach Anspruch 4 oder 5, wobei das Statussignal ein elektrisches Signal umfasst und wobei die Lichtquelle (102, 302, 402, 502) dazu eingerichtet ist, zur Erzeugung des Lichtsignals das elektrische Signal zu verwenden.

7. Visueller Statusanzeiger (200) nach Anspruch 4 oder 5, wobei das Statussignal ein Lichtsignal umfasst und wobei die Lichtquelle (202) dazu eingerichtet ist, das Lichtsignal auszustrahlen.

8. Visueller Statusanzeiger (200) nach Anspruch 7, wobei der Statuseingang einen ersten Bereich (204) eines Lichtleiters (203) umfasst, wobei die Lichtquelle einen zweiten Bereich (202) des Lichtleiters umfasst, wobei das Lichtsignal von dem ersten Bereich zu dem zweiten Bereich des Lichtleiters geleitet wird und das Lichtsignal aus dem zweiten Bereich abgestrahlt wird.

9. Visueller Statusanzeiger (400, 500) nach einem der vorhergehenden Ansprüche, wobei der gemessene Status dem Durchflussvolumenstrom oder der Volumenstromrichtung des Fluids (111) zugeordnet ist und wobei die Lichtquelle (402, 502) dazu eingerichtet ist, die Position des Lichtsignals in Abhängigkeit von dem Durchflussvolumenstrom oder der Volumenstromrichtung zu variieren, um eine sich bewegende visuelle Statusanzeige des Durchflussvolumenstroms oder der Volumenstromrichtung zu erzeugen.

10. Visueller Statusanzeiger (100, 200, 300) nach einem der Ansprüche 1 bis 8, wobei der gemessene Status mit der Zusammensetzung, der Konzentration oder dem Druck des Fluids (111) zusammenhängt und die Lichtquelle dazu eingerichtet ist, in Abhängigkeit von dem gemessenen Status eine Farbe oder eine Intensität des Lichtsignals zu variieren.

11. Visueller Statusanzeiger (300, 400) nach einem der vorhergehenden Ansprüche, wobei die Befestigungsmittel (301, 401) dazu ausgestaltet sind, um den visuellen Statusanzeiger (300, 400) wenigstens entlang eines Bereichs der Patientenleitung (310, 410) zu befestigen, der dem Patienten (130) näher liegt als dem medizinischen Gerät (120).

12. Beatmungsschlauch (410, 510) zum Transportieren eines Fluids (111) zwischen einem medizinischen Gerät (120) und einem Patienten (130), wobei der Beatmungsschlauch den visuellen Statusanzeiger nach einem der vorhergehenden Ansprüche umfasst.

13. Beatmungsschlauch (410, 510) nach Anspruch 12, wobei der visuelle Statusanzeiger (400, 500) integraler Bestandteil des Beatmungsschlauchs ist.

14. Medizinisches Gerät (120) zum Transportieren eines Fluids (111) durch eine Patientenleitung (110), wobei das medizinische Gerät einen Geräteanschluss (122) aufweist, der es erlaubt, dass die Patientenleitung und der visuelle Statusanzeiger nach Anspruch 5 gleichzeitig mit dem medizinischen Gerät verbunden sind, und wobei der Geräteanschluss einen Fluidanschluss (123) zum Liefern des Fluids zu der Patientenleitung und eine Statusausgabe (121) aufweist, um dem visuellen Statusanzeiger (100) ein Statussignal zu liefern, das repräsentativ für einen gemessenen Status des Fluids (111) ist.

## Revendications

1. Indicateur d'état visuel (100, 200, 300, 400, 500) pour fournir une indication d'état d'un fluide (111) transporté à travers une tubulure patient. (110, 310, 410, 510) entre un dispositif médical (120, 220) et un patient (130), l'indicateur d'état visuel comprenant :
- un moyen de fixation (301, 401) pour fixer l'indicateur d'état visuel le long d'une partie de la tubulure patient, et
- une source lumineuse (102, 202, 302, 402, 502) pour fournir au niveau de ladite partie un signal lumineux en fonction d'un état mesuré du fluide transporté à travers la tubulure patient,
dans lequel une partie de la tubulure patient comprend un tube de respirateur (410),
**caractérisé en ce que**
le moyen de fixation comprend un manchon (401) pour s'adapter au moins partiellement autour du tube de respirateur.

2. Indicateur d'état visuel (100, 200, 300, 400, 500) selon la revendication 1, dans lequel la source lumineuse (102, 202, 302, 402, 502) est conçue pour faire varier une caractéristique visuelle du signal lumineux en fonction de l'état mesuré du fluide (111), la caractéristique visuelle comprenant au moins un élément parmi la couleur, l'intensité et la position du signal lumineux.

3. Indicateur d'état visuel (100, 200, 300, 400, 500) selon la revendication 1 ou 2, dans lequel l'état mesuré du fluide (111) comprend au moins un élément parmi le débit, la direction d'écoulement, le changement de débit, le changement de direction d'écoulement, la pression, la composition du fluide et la concentration du fluide.

4. Indicateur d'état visuel (100, 200, 300, 400, 500) selon l'une quelconque des précédentes revendications, l'indicateur d'état visuel comprenant une entrée d'état (104, 204, 304, 404, 504) pour recevoir en provenance du dispositif médical (120, 220) un signal d'état représentatif de l'état mesuré du fluide (111), et la source lumineuse (102, 202, 302, 402, 502) étant conçue pour fournir le signal lumineux en fonction en fonction du signal d'état.

5. Indicateur d'état visuel (100, 200, 400, 500) selon la revendication 4, dans lequel l'entrée d'état (104, 204, 404, 504) de l'indicateur d'état visuel est conçue pour former, conjointement avec un connecteur de fluide (506) de la tubulure patient, un connecteur (505) qui permet à l'indicateur d'état visuel et à la tubulure patient d'être simultanément reliés au dispositif médical.

6. Indicateur d'état visuel (100, 300, 400, 500) selon la revendication 4 ou 5, dans lequel le signal d'état comprend un signal électrique, et la source lumineuse (102, 302, 402 502) est conçue pour générer le signal lumineux au moyen du signal électrique.

7. Indicateur d'état visuel (200) selon la revendication 4 ou 5, dans lequel le signal d'état comprend un signal lumineux, et la source lumineuse (202) est conçue pour émettre le signal lumineux.

8. Indicateur d'état visuel (200) selon la revendication 7, dans lequel l'entrée d'état comprend une première partie (204) d'un conducteur de lumière (203), la source lumineuse comprenant une seconde partie (202) du conducteur de lumière, le signal lumineux étant transporté de la première partie à la seconde partie du conducteur de lumière et le signal lumineux étant émis depuis la seconde partie.

9. Indicateur d'état visuel (400, 500) selon l'une quelconque des précédentes revendications, dans lequel l'état mesuré est associé à un débit ou à une direction d'écoulement du fluide (111), et la source lumineuse (402, 502) est conçue pour faire varier, en fonction du débit ou de la direction d'écoulement, une position du signal lumineux afin de créer une indication d'état visuelle mobile du débit ou de la direction d'écoulement.

10. Indicateur d'état visuel (100, 200, 300) selon l'une quelconque des revendications 1 à 8, dans lequel l'état mesuré est associé à une composition, une concentration ou une pression du fluide (111), et la source lumineuse (102, 202, 302) est conçue pour faire varier, en fonction de l'état mesuré, une couleur ou une intensité du signal lumineux.

11. Indicateur d'état visuel (300, 400) selon l'une quelconque des précédentes revendications, dans lequel le moyen de fixation (301, 401) est prévu pour fixer l'indicateur d'état visuel (300, 400) le long d'au moins une partie de la tubulure patient (310, 410) qui est située plus près du patient (130) que le dispositif médical (120).

12. Tube de respirateur (410, 510) pour transporter un fluide (111) entre un dispositif médical (120) et un patient (130), le tube de respirateur comprenant l'indicateur d'état visuel selon l'une quelconque des précédentes revendications.

13. Tube de respirateur (410, 510) selon la revendication 12, dans lequel l'indicateur d'état visuel (400, 500) fait partie intégrante du tube de respirateur.

14. Dispositif médical (120) pour transporter un fluide (111) à travers une tubulure patient (110), le dispositif médical comprenant un connecteur de dispositif (122) qui permet à la tubulure patient et à l'indicateur d'état visuel de la revendication 5 d'être reliés simultanément au dispositif médical, et le connecteur de dispositif comprenant un connecteur de fluide (123) pour fournir le fluide à la tubulure patient et une sortie d'état (121) pour fournir à l'indicateur d'état visuel (100) un signal d'état représentatif d'un état mesuré du fluide (111).
